# EUROPEAN PATENT SPECIFICATION

(11) **EP 2 349 417 B1**
(45) Date of publication and mention of the grant of the patent: **30.03.2016**
(21) Application number: 09752450.8
(22) Date of filing: 09.11.2009
(51) Int. Cl.: A61M 15/00

(54) **INHALER DEVICE**
INHALATOR
DISPOSITIF INHALATEUR

(43) Date of publication of application: 03.08.2011
(73) Proprietor: Medispray Laboratories Pvt.,Ltd., Ponda 403115 GOA (IN)
(72) Inventor: SAWANT, Rajesh, Navi Mumbai 400 709 (IN)
(74) Representative: Bennett, Adrian Robert J.
(86) International application number: PCT/GB2009/002635
(87) International publication number: WO 2010/052479

(56) References cited:
- EP-A1- 2 082 763
- EP-A2- 1 917 992
- WO-A1-2004/103446
- WO-A2-2008/053253
- WO-A2-2008/114034
- GB-A- 2 447 560

## Description

The present invention relates to inhalers for the administering of medicament. The invention relates particularly to inhalers which are used with piercible sealed compartments of medicament and, more particularly, to inhalers for receiving cartridges having a plurality of such compartments. Such inhalers allow for the administration of predetermined doses of medicament (typically a dry powder medicament).

A wide variety of inhalers are presently available on the market, which are designed to receive one or more sealed compartments of medicament. When medicament in a particular compartment is required for inhalation by a user of the inhaler device, a piercing member is used to pierce a seal of the compartment and extend through said seal into proximity with the medicament contained within the compartment. The medicament may then be inhaled through a flow path extending through the piercing member into the compartment.

A problem associated with inhalers of the above type is that a rapid inhalation of air (and medicament) through said flow path in the piercing member tends to be difficult for a user.

A prior art inhaler according to the preamble of the appended claims is disclosed in WO 2008/114034.

It is an object of the present invention to provide an inhaler device which allows air and medicament to be inhaled more readily and rapidly.

The invention is defined by independent cliam 1.

A first aspect of the present invention provides an inhaler device for dispensing doses of medicament, the inhaler comprising means for receiving a compartment containing medicament; and medicament extraction facilitating means for locating in a received compartment and thereby allowing an extraction of medicament from said compartment; wherein the extraction facilitating means comprises (i) a first fluid pathway extending therethrough for directing extracted medicament to an outlet of the inhaler and (ii) means which, in use, provides a second fluid pathway for directing fluid into said compartment past a sealing material of the compartment pierced by said extraction facilitating means.

The extraction facilitating means ideally comprises an element for piercing a sealing material of a received compartment, said element comprising a first part provided with said first fluid pathway therein and a second part providing said second fluid pathway and wherein the second part ideally provides said second fluid pathway as a pathway extending through said part and the extraction facilitating means further comprises a ridge for piercing a sealing material of a compartment, and wherein said piercing ridge extends from the first part to the second part. The second part may be an element extending laterally of the first part which, in use, spaces pierced sealing material from the first part.

Preferably, the second part extends laterally from the first part and is connected thereto. The second part may be a planar member.

The second part may comprise an inlet aperture at an end thereof distal to an opposite end thereof which, in use, locates in a received compartment.

In an extraction position of the medicament extraction facilitating means, the inlet aperture of the second part preferably locates on one side of pierced scaling material external to the compartment and said opposite end of the second part ideally locates on the other side of the pierced sealing material internally within the compartment.

Ideally, the second part comprises an outlet aperture at said opposite end thereof.

The piercing ridge may be an apex formed by the meeting of two surfaces.

The inhaler device may be for dispensing multiple doses of medicament, the inhaler comprising means for receiving a medicament cartridge comprising a plurality of compartments containing medicament; and operating means moveable in first and second directions by a user; wherein the medicament extraction facilitating means is movable from a retracted position to an extraction position in response to movement of the operating means in the first direction, the extraction position of the medicament extraction facilitating means allowing extraction of medicament from said cartridge compartment upon inhalation by a user, and the retracted position allowing advancement of the medicament cartridge, and wherein the medicament cartridge is driven by the operating means so as to advance a cartridge compartment into a predetermined position relative to the medicament extraction facilitating means in response to movement of the operating means in the second direction.

The inhaler device may comprise medicament extraction facilitating means comprising a resiliently displaceable member.

The inhaler device may comprise medicament extraction facilitating means wherein said means is sprung biased or is operated by a cam mechanism and displaced over a pivot. Ideally, said means is biased into a medicament extraction position allowing extraction of medicament from a medicament cartridge. Preferably said means is sprung biased by virtue of the resiliently deflectable character of the material from which said means is manufactured.

Preferably, the medicament extraction facilitating means moves from the extraction position to the retracted position in response to movement of the operating means in the second direction.

The medicament extraction facilitating means may preferably be formed from acetal, but ABS, nylon, polycarbonate, HDPE or LDPE may also be used.

It will be understood therefore that an inhaler device according to the present invention allows for a comparatively simple operation by a user through appropriate movement of the operating means. If a user wishes to inhale a dose of medicament from a cartridge within the inhaler device, then the user need only move the operating means in a first direction so as to move the extraction facilitating means to an extraction position in which medicament may be extracted from the cartridge compartment. A user may then readily move the operating means in a second direction so as to advance or index the next dose of medicament to an appropriate position. Typically, this position will be one adjacent to the piercing end of the medicament extraction facilitating means. The medicament extraction facilitating means may be moved to a retracted position which allows for the advancement of the medicament cartridge. It will be appreciated that the operating means may drive both the advancement of the cartridge and movement of the medicament extraction facilitating means.

Whilst the operating means drives the medicament extraction facilitating means to one of the extraction and retracted positions, biasing means may be provided for moving the medicament extraction facilitating means to the other of the extraction and retracted positions. The biasing means may comprise a resilient elastic member. The resilient elastic member may be provided as a cantilever arm, with one end thereof fixed to a housing of the inhaler device and a free end thereof being provided with the medicament extraction facilitating means.

The operating means may, alternatively, drive the medicament extraction facilitating means to both the extraction and retracted positions. Biasing means may also be provided to bias the medicament extraction facilitating means towards one of the extraction and retracted positions.

The operating means may drive the medicament extraction facilitating means through use of a cam arrangement. Ideally, a cam is provided on the operating means. The cam may be provided as a ramp surface. The arrangement may be such that, when the operating member is moved in the second direction, the ramp surface moves laterally in abutment with the medicament extraction facilitating means and thereby displaces the medicament extraction facilitating means. The medicament extraction facilitating means may be provided with a roller or bearing before abutment with the cam.

The medicament extraction facilitating means may be provided with a tubular element for locating in a cartridge compartment when the medicament extraction facilitating means is located in the extraction position. The cross-section of the tubular element may have a circular, oval, square or rectangular shape. A free end of the tubular element may be provided with a piercing portion. The piercing portion may be of a frusto-conical shape. Alternatively, the piercing portion may have the shape of a triangular based pyramid or prism. Furthermore, the arrangement of the medicament extraction facilitating means may be such that, when the medicament extraction facilitating means is located in the extraction position, the tubular element is in fluid communication with a mouthpiece of the inhaler device. This fluid communication may be made through an aperture or discontinuity in the operating means. This aperture or discontinuity is ideally aligned with a flow path through the mouthpiece when the operating means has been moved in the first direction so as to drive the medicament extraction facilitating means into the extraction position.

The mouthpiece may also include an opening (through which medicament is inhaled) which is provided with a filter for deaggregation of the medicament/fluid to be inhaled and/or helical guide vanes for imparting a swirl motion onto medicament/fluid to be inhaled. Other means for imparting a swirl motion on medicament/fluid may be provided.

It is also preferable for the operating means to close a flow path through the mouthpiece when the operating means is moved in the second direction. Ideally, the aperture or discontinuity is moved out of alignment with the mouthpiece flow path when the operating means is moved in the second direction. Fluid communication between the mouthpiece and the extraction facilitating means is ideally prevented when the medicament extraction facilitating means is in the retracted position.

Furthermore, the medicament extraction facilitating means may optionally comprise a venturi.

It is also desirable for ratchet means to be provided for allowing the operating means to drive the medicament cartridge. The operating means may be provided with a ratchet tooth so that the medicament cartridge is moved in response to movement of the operating means in the second direction, but is not moved in response to movement of the operating means in the first direction. Said ratchet tooth may be arranged so as to engage in turn with each of a plurality of ratchet teeth provided on the medicament cartridge.

Ideally, said first direction is opposite to said second direction. It is also preferable for movement of the operating means in said first and second directions to comprise a movement of the operating means along a part-circular path. The part-circular path is ideally concentrically positioned relative to an annular arrangement of medicament compartments of the medicament cartridge.

An element of the operating means may extend through a slot in a housing of the inhaler device. The slot is ideally configured so as to allow movement of said element between opposite ends of the slot and thereby move the operating means in said first and second directions. Retaining means may be provided for preventing movement of said element of the operating means. Said retaining means may be provided on a cover for the mouthpiece, possibly as part of a cover for the mouthpiece.

The inhaler device may be provided with means for retaining a medicament cartridge in a required position. The retaining means may comprise a roller, wheel, bearing or cam to press against the cartridge whilst allowing movement of the cartridge. The retaining means may be sprung biased so as to press against the medicament cartridge and/or between teeth of the cartridge. The retaining means may be made from acetal, but may be made from ABS, nylon, polycarbonate, HDPE or LDPE. The inhaler may also comprise means for driving a dose counter in response to movement of a medicament cartridge. Said drive means may comprise one or more teeth or detents for engaging a medicament cartridge. The drive means may also comprise a gear wheel for engaging means for displaying a dose count.

Embodiments of the present invention will now be described with reference to the accompanying drawings, in which:
Figure 1 is a perspective view of the exterior of an inhaler device according to the present invention;
Figure 2 is a top view of the exterior of the inhaler device shown in Figure 1;
Figure 3 is a bottom view of the exterior of the inhaler device shown in Figure 1;
Figure 4 is a side view of the exterior of the inhaler device shown in Figure 1;
Figure 5 is a perspective view of the exterior of the inhaler device of Figure 1 shown in an operative position wherein a spiking component within the inhaler is piercing a sealed compartment of medicament;
Figure 6 is a perspective view of a base of the inhaler device of Figure 1, with a medicament cartridge and internal components of the inhaler device mounted on said base;
Figure 7 is a perspective view of a body (or middle compartment) of the inhaler device with internal components of the inhaler device mounted on said middle cover;
Figure 8 is an enlarged view of the spike component mounted on said middle cover;
Figure 9 is a perspective view of a base of a modified inhaler device, with a medicament cartridge and internal components of the modified inhaler device mounted on said base;
Figure 9A is a perspective view of a modified deformable member shown in Figure 9;

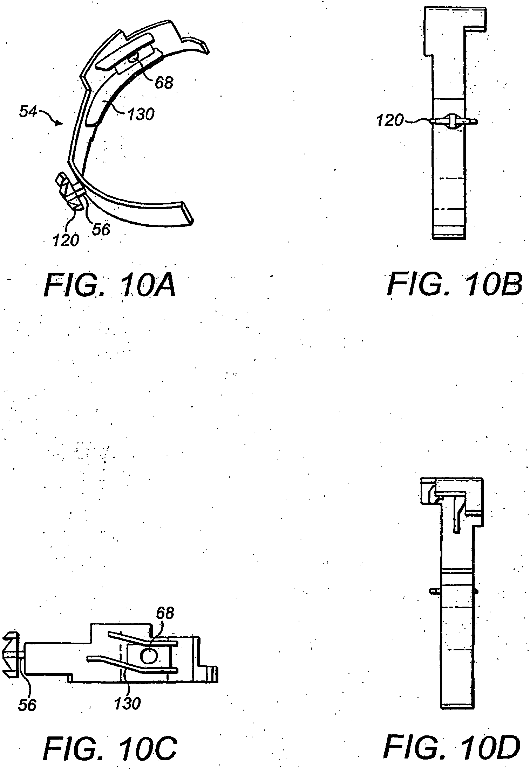

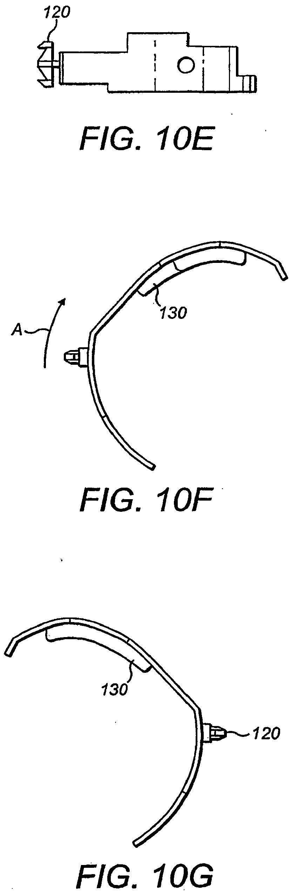

Figures 10A-G are perspective, front, right side, rear, left side, top and bottom views respectively of an operating member of the inhaler device;
Figure 11 is a perspective view of a first type of spiking component;
Figure 12 is a perspective view of a second type of spiking component; and
Figure 13 is a side view of a third type of spiking component.

An inhaler device 1 is shown in the accompanying drawings which comprises an outer housing and a plurality of internal components including a cartridge having a plurality of compartments containing medicament. Amongst the internal components are an arm 90 mounted to the housing, a spiking component 22 mounted to the arm 90, a dose counter mechanism, and an operating member 54.

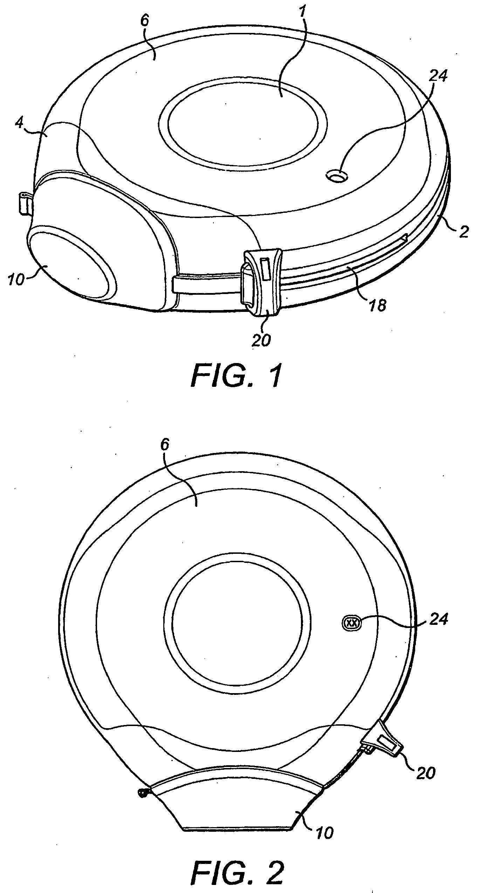

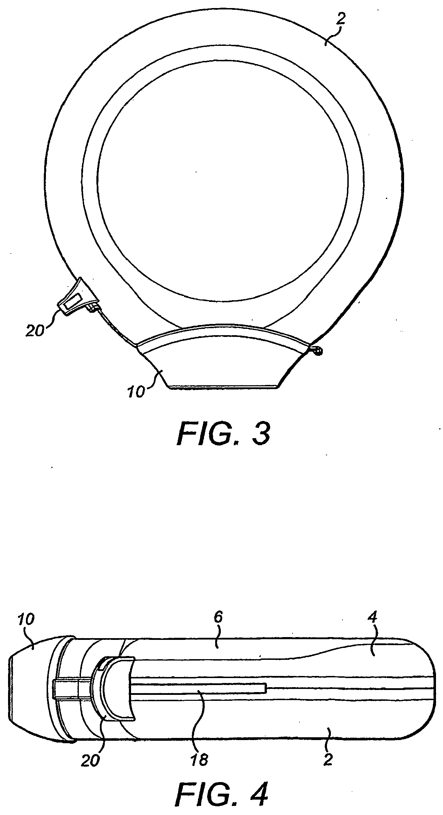

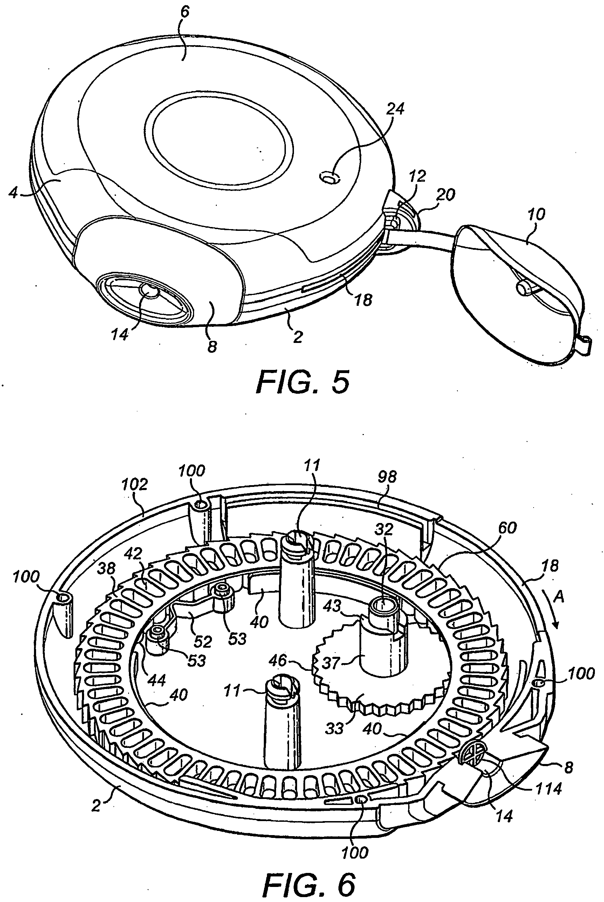

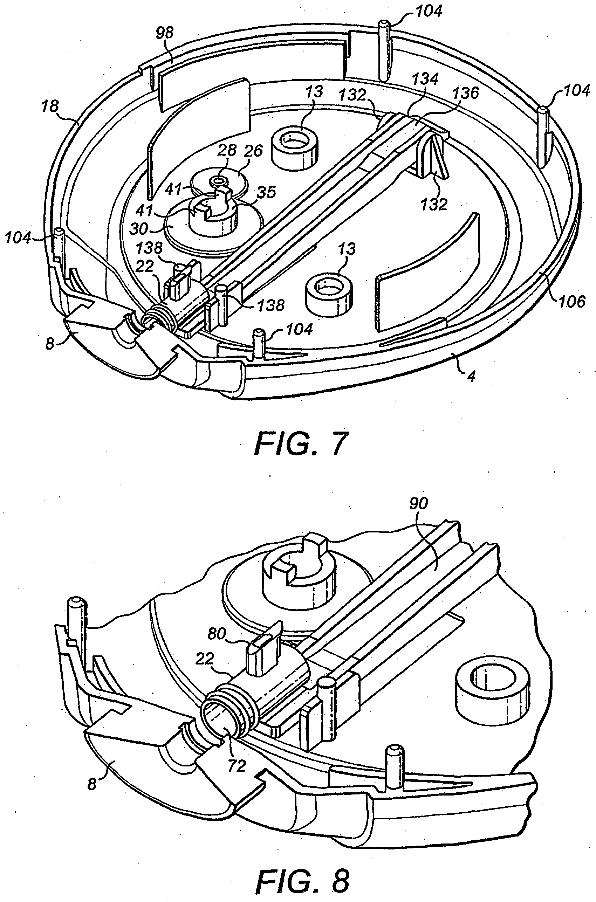

The housing comprises a base 2, a middle cover 4, and a top cover 6 (see Figures 1 to 5). Four apertures 100 are provided along an upper perimeter edge 102 of the base 2 (see Figure 6) in order to receive four posts 104 extending downwardly from a lower perimeter edge 106 of the middle cover 4 (see Figure 7). The apertures 100 are asymmetrically positioned along the perimeter edge 102 of the base 2 and the posts 104 are arranged in corresponding positions along the perimeter edge 106 of the middle cover 4 so that all four posts 104 can only be simultaneously inserted within the apertures 100 when the base 2 and middle cover 4 are correctly aligned in a particularly required position relative to one another. With the base 2 and middle cover 4 correctly aligned, two further posts 11 upstanding from the interior surface of the base 2 snap fit into two cylindrical bosses 13 upstanding from the interior surface of the middle cover 4. The base 2 and middle cover 4 are thereby secured to one another. The top cover 6 is attached to the middle cover 4 using an arrangement of posts and which are retained in corresponding apertures. Other means of attachment may be used.

The housing is provided with a mouthpiece 8 and a mouthpiece cover 10. An aperture 14 in the front side of the housing allows for fluid communication through the mouthpiece 8 between the interior and exterior of the inhaler housing. The aperture 14 is optionally provided with a mesh 114 for deaggregation of the medicament particles prior to inhalation. In use, a user of the inhaler device 1 places the mouthpiece to the lips of their mouth and inhales through the mouthpiece so as to extract and inhale medicament.

In an alternative embodiment, the mouthpiece 8 may be replaced with a construction suitable for nasal inhalation.

One or more further apertures may be provided in the rear of the housing, which assists with allowing a flow of air into the housing during inhalation of medicament by a user.

The housing is also provided with a slot 18 along which a knob 20 may be moved so as to advance a cartridge of medicament within the housing and so as to displace a spiking component 22. The mouthpiece cover 10 is pivotally arranged relative to the knob by means of a hinge arrangement 12. The cover 10 and knob 20 may be an integral unitary component with the hinge arrangement comprising a resilient flexible element, or the cover 10 and knob 20 may be separate components with the hinge arrangement allowing said separate components to be connected to one another in such a way as to allow a relative rotation between them. In this latter case, the hinge arrangement may provide for the two components to be snap-fitted to one another, for example, by the location of two opposing bosses on one of said components into two opposing apertures or recesses in the other of said components.

The coupling of the cover 10 and the knob 20 allow them to be conveniently moved together during actuation of the device. In particular, a single hand can be used to open the mouthpiece cover 10 and actuate the device by moving the knob 20.

An aperture or window 24 is also provided in the middle cover 4 and the top cover 6 so that an indication of the number of doses used or the number of doses remaining can be seen provided on a dose counter wheel 26 located within the housing.

The counter wheel 26 is located on and rotatably attached to a first boss 28 upstanding from an interior surface of the middle cover 4. A drive wheel 30 for rotating the counter wheel 26 is located adjacent said interior surface of the middle cover 4 and is rotatably mounted to a second boss 32. The second boss (provided as a cylindrical member) upstands from an interior surface of the base 2 and, in the assembled inhaler device, extends to a position proximate said interior surface of the middle cover and is positioned relative to the first boss 28 so that the drive wheel 30 drivingly engages with the counter wheel 26. This engagement may be, for example, by means of gear teath (not shown) or by friction alone.

A drive gear 33 is also rotatably mounted on the second boss 32 and is located adjacent said interior surface of the base 2 from which the second boss 32 upstands. The drive wheel 30 and drive gear 33 are connected to one another by means of an axle connected to the centre of said drive wheel 30 and gear 33. The axle is provided as a two-part cylindrical shaft, which receives the second boss 32 within its interior and extends along the length of the second boss 32. A first part 35 of the axle is connected to the drive wheel 30 and a second part 37 of the axle is connected to the drive gear 33. The two parts 35,37 are connected to one another by means of mating castellations 41,43 provided on the ends of the two parts 35,37 distal to the the drive wheel 30 and drive gear 33 respectively. Alternative means for connecting the two parts 35,37 may be used.

In use, the drive gear 33 is rotated by the rotation of a medicament cartridge 38 and, due to the engagement of the castellations 41,43 on the two parts of the axle, the drive gear 33 rotates the drive wheel 30 which, in turn, rotates the counter wheel 26. In this way, an appropriate indicator provided on the upperside of the counter wheel 26 may be viewed through the window 24 and thereby provide a user with an indication of the remaining doses or the doses used.

Three equi-spaced support members 40 are provided on the interior surface of the base 2 to ensure a medicament cartridge 38 locates and rotates correctly adjacent said surface. The support members 40 are part-circular and positioned on the circumference of the same circle. The medicament cartridge 38 locates below the counter wheel 26 and drive wheel 30 but in the same plane as the drive gear 33.

The cartridge 38 is provided as an annular ring having a plurality of compartments 42. Specifically, the cartridge 38 has sixty to seventy compartments. Each compartment contains medicament in a powder form. The annular ring comprises two operative walls - a radially outer operative wall and a radially inner operative wall. Both walls are provided with a plurality of teeth. The inner wall comprises a plurality of integral teeth 44, which engage with a plurality of teeth 46 provided on the perimeter of the drive gear 33. The arrangement is such that a small number of teeth 46 are engaged at any one time with the same number of teeth 44 of the medicament cartridge 38. Thus, as the cartridge 38 rotates (with the inner cartridge wall abutted and guided by the three support members 40), the drive gear 33 is rotated on the boss 32 by virtue of the engagement between the aforesaid teeth 44, 46.

Rotary movement of the medicament cartridge 38 is resisted by means of a resiliently deformable member 52, which is sprung biased so as to be press into abutment with the radially inner wall of the cartridge 38 between the teeth 44 thereof. The deformable member 52 comprises an element for locating between said teeth 44. The element has a V-shape. The deformable member 52 further comprises two legs, each leg extending from a side of said element and having an aperture therein which is fitted onto one of two posts 53 upstanding from the base 2. The posts 53 have a flange (not shown) provided on their free ends so as to retain the deformable member 52 on the base 2.

The cartridge 38 will only rotate if sufficient force is applied to the cartridge 38 to overcome the bias of said deformable member 52 and thereby slide the deformable member 52 over the teeth 44. In this way, undesirable rotation of the medicament cartridge 38 is prevented and the cartridge itself is always biased towards one of a plurality of predetermined rotary positions.

The medicament cartridge 38 is moved from one medicament compartment 42 to the next, during use, by moving an operating member 54. An element 56 of the operating member 54 extends through the slot 18 and attaches with the knob 20. The element 56 is provided with a clip 120 for attaching the element 56 to the knob 20. The operating member 54 comprises a ratchet tooth, which engages with a plurality of ratchet teeth 60 provided on the outer operative wall of the medicament cartridge 38. The configuration and engagement of the ratchet teeth 60 is such that, when the operating member 54 is moved in the direction of arrow A (see Figure 6) the ratchet tooth of the operating member 54 abuts against an adjacent ratchet tooth 60 of the cartridge 38 and rotates the cartridge 38 in the direction of arrow A. The extent of movement of the operating member 54 allowed by the slot 18 indexes the cartridge 38 by one compartment 42 (i.e. to the next compartment 42). The movement of the cartridge 38 will also be understood to drive the dose counter wheel 26 as already described above.

When the operating member 54 is moved in an opposite direction to that indicated by arrow A, the configuration of the ratchet mechanism allows the ratchet tooth on the operating member 54 to ride over the ratchet teeth 60 of the cartridge 38 without exerting sufficient force to overcome the bias of the deformable member 52. The cartridge 38 is therefore not rotated by movement of the operating member 54 in a direction opposite to that indicated by arrow A.

The operating member 54 further comprises a sealing face plate which aligns with the flow path 14 associated with the mouthpiece 8 when the operating member 54 has been moved to the fullest extent in the direction indicated by arrow A. Furthermore, the operating member 54 is provided with an aperture 68 which aligns with the flow path 14 when the operating member 54 has been moved to the fullest extend in the direction opposite to that indicated by arrow A.

The operating member 54 yet further comprises a camming member/strip or ramp, which engages with an end portion 72 of a spiking component 22. The end portion may be provided with a roller to assist with the camming action of the camming member. In the assembled inhaler 1, the camming strip is located above said end portion 72 and the arrangement is such that, when the operating member 54 is moved in a direction opposite to that indicated by arrow A, the end portion 72 slides against the underside of the cam strip and is pressed downwardly by the camming strip 70 so as to locate the spiking component within a previously advanced compartment 42 of medicament. In so locating the spiking component 22, a seal provided across the top of the compartment 42 is pierced/perforated by the spiking component 22.

The spiking component 22 comprises a perforating element 80, the interior of which provides fluid communication between a compartment (in which said component 22 locates) and the aperture 68 and flow path 14. Thus, with the operating member 54 having been moved in a direction opposite to that indicated by arrow A, the perforating element 80 is located in a compartment containing medicament and the aperture 68 of the operating member 54 is aligned with the mouthpiece flow path 14. Medicament may then be inhaled from the medicament compartment from the mouthpiece.

The operating member 54 is also provided with a second camming strip 130 located below the aperture 68. This second cam 130 is orientated so as to press the end portion 72 of the spiking component 22 upwardly thereby returning the support arm 90 to an upper position within the housing (as the operating member 54 is moved in a direction as indicated by arrow A). In this way, the arm 90 is returned to its storage/retracted position by means of a camming action.

When the operating member 54 is moved back in the direction indicated by arrow A, the aperture 68 is moved out of alignment with the mouthpiece flow path 14. The flow path 14 is then covered by the sealing plate 66 of the operating member 54. Also, as the operating member 54 is moved in the direction of arrow A, the downward pressure applied by the cam 70 is released to allow the spiking component 22 to be moved upwardly by the second cam 130.

The arm 90 is secured to the middle cover 4 at an opposite end of the arm 90 to the end portion 72. The arm 90 is pivotally connected as a cantilever to the middle cover 4. This pivotal connection allows the arm 90 to move to and from the retracted/storage position without a bending or other deformation of the arm 90. The arm 90 is therefore resiliently displaceable.

The pivotal connection of the arm is provided by resilient clip elements 132 projecting downwardly from the underside of the middle cover 4. These clip elements 132 are provided with bosses 134 (only one of which is partially visible in Figure 7) for snap-fitting into apertures 136 (again, only one of which is partially visible in Figure 7) of the arm 90. The arm 90 is rotatable up and down about the bosses 134. When the arm 90 is located in its upper-most position within the housing of the inhaler device 1, the arm 90 locates between two elements 138 projecting downwardly from the underside of the middle cover 4. In this way, the end of the arm 90 distal to the apertures 1336 is prevented from moving from side to side.

The arm 90 is not biased to a particular position, however such a bias may be provided, if considered desirable, through use of a spring.

It will be understood that sliding the operating member 54 in the direction of arrow A results in moving the inhaler 1 into a closed position, whilst sliding the operating member 54 in the direction opposite to that indicated by arrow A results in moving the inhaler 1 into an open position.

The operating member 54 is located within the inhaler housing in a groove 98. The groove 98 allows the operating member 54 to be displaced along the inner side of the housing (i.e. circumferential movement) whilst limiting undesirable radial movement.

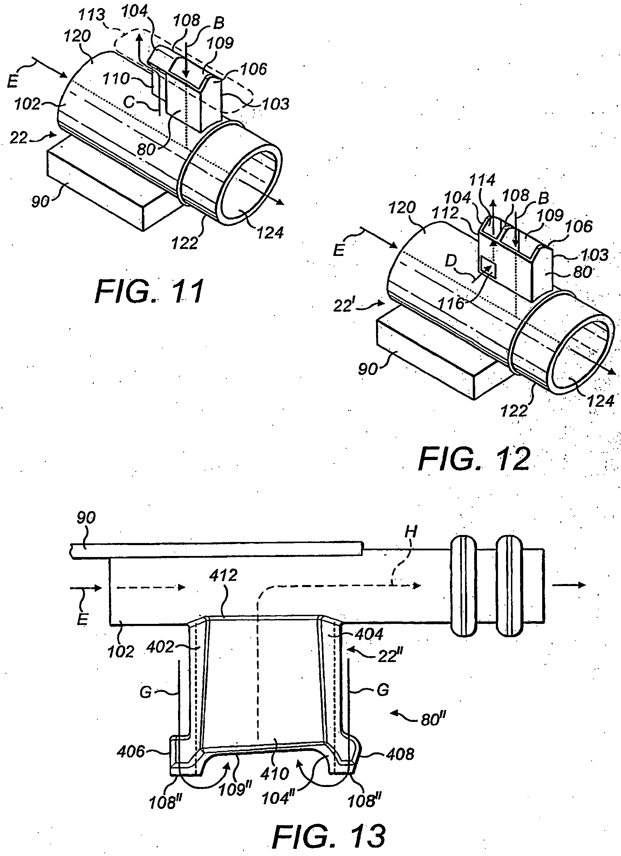

An enlarged view of the spiking component 22 is shown in Figure 11 of the accompanying drawings. The spiking component 22 comprises a cylindrical member 102 defining a fluid pathway therein. A spiking element 80 extends perpendicularly from the exterior of the cylindrical member 102. A first part 103 of the spiking element 80 has a rectangularly shaped cross-section. The free end of the spiking element 80 is provided with two sloping surfaces 104,106 which extend from the major sides (as opposed to minor sides) of the rectangular first part 103 cross-section and meet one another at an apex forming a ridge 108. The ridge 108 is therefore parallel with the major sides (as opposed to minor sides) of the rectangular first part 103 cross-section. In use, the ridge 108 pierces/perforates the seal of a medicament compartment.

The first part 103 of the spiking element 80 is provided with a flow pathway (of rectangular cross-section) extending therethrough. This flow pathway opens onto the sloping surfaces 104,106 to form an aperture 109 therein and is in fluid communication with the fluid pathway extending through the cylindrical member 102. In use, medicament is extracted from a pierced compartment by inhalation breath through the aperture 109 in the direction indicated by arrow B.

The ridge 108 and, therefore, the sloping surfaces 104,106 extend beyond the first part 103 in a cantilever fashion. The extended sloping surfaces 104,106 are supported by means of a second part 110 in the form of a wall extending perpendicularly from the exterior of the cylindrical member 102 to beneath the ridge 108. The wall is oriented parallel with the major of the rectangular first part 103 cross-section and the ridge 108. The thickness of the wall is less than the width of the first part 103 (i.e. less than the length of the minor of the rectangular first part 103 cross-section). As a consequence, a space is provided either side of the second part 110 between the extended sloping surfaces 104,106 and the cylindrical member 102. During use, when the spiking element 80 is located in a pierced compartment, the spiking element 80 (and, in particular, the first and second parts 103,110) push to one side material of the compartment which had previously sealed said compartment. The space either side of the second part 110 then allows inhalation breath to flow readily into the pierced compartment (rather than being restricted from doing so by sealing material). This flow of inhalation breath is indicated by arrow C in Figure 11 and the position of the compartment relative to the spiking component is shown by dotted line 113. It will be seen that the inhalation breath flows into said space, over the edges of the extended sloping surfaces 104,106 and then over the medicament (which is now located between the sloping surfaces 104,106 and the bottom of the compartment). Medicament thereby becomes entrained into the air inhalation flow and passes from the compartment, via the aperture 109 in the sloping surfaces 104,106. as indicated by arrow B. Also, the proximity of the sloping surfaces 104,106 to the bottom of the compartment reduces the likelihood of the air flow to re-circulate repeatedly within the compartment rather than immediately flowing out of the compartment. As a result, a user may breath more readily through the inhaler device and receive a full dose of medicament more rapidly and efficiently. This is also assisted, in part, by the free end of the spiking element 80 having a similar plan shape (rectangular) and size to that of the medicament compartments.

It will be understood that alternative means may be provided for allowing air to flow more readily into a pierced compartment and/or for reducing the effective volume within a compartment to thereby reduce a tendency for recirculation. For example, a second spiking component 22' is shown in Figure 12. The second spiking component 22' is very similar to the first spiking component 22, and like elements are identified in the drawings with like reference numerals. However, the second spiking component 22' is different from first spiking component 22 in that the wall of the second part 110 is replaced with a rectangular box section 112 extending perpendicularly from the exterior of the cylindrical member 102 to the extended sloping surfaces 104,106. This rectangular box section 112 defines a second flow pathway (of rectangular cross-section) extending through the spiking element 80 and opening onto the extended sloping surfaces 104,106 by means of an outlet aperture 114 in said extended surfaces. An inlet aperture 116 is provided in the box section 112 adjacent the cylindrical member 102 so as to allow inhalation breath to flow into a pierced compartment via said second flow pathway and the outlet aperture 114, as indicated by arrow D in Figure 12. The arrangement of the inhaler is such that, when the spiking element 80 is located in a pierced compartment, the pierced sealing material locates between the free end of the spiking element 80 (i.e. the free end of the box section 114) and the inlet aperture 116. In this way, air flowing into the pierced compartment bypasses the pierced sealing material by flowing into the inlet aperture 116, along the pathway of the box section 112, and out of the outlet aperture 114. The sealing material does not therefore then present an obstruction to the flow of air into the compartment.

It should be noted that the cylindrical member 102 does not open into the box section 112. The box section 112 is in fluid communication with the interior of the cylindrical member 102 only by means of the first part 103.

The fluid pathway in the cylindrical member 102 extends from a first end 120 of said member 102 to a second end 122 thereof. The fluid pathway in the cylindrical member 102 may comprise a venturi (a restriction followed by an expansion of the pathway) which is positioned adjacent the fluid pathway of first part 103 so that the consequential reduction in static pressure of the inhalation air flowing from the first end 120 to the second end 122 (indicated by arrow E) encourages medicament/air to travel up the fluid pathway of first part 103 and into the venturi before eventually entering the lungs of the user. Fluid communication is provided between the fluid pathway of first part 103 and an outlet 124 of said second end 122.

In each spiking component 22,22' the first and second parts 103,110,112 and the sloping surfaces 104,106 may be a single unitary component. It will be understood that the sloping surfaces may be replaced with a non-sloping surface. However, the seal piercing/perforating ridge will then no longer be present and the piercing/perforating performance of the spiking component may be adversely affected.

A further spiking component 22" is shown in Figure 13 of the accompanying drawings. This alternative spiking component comprises a similar cylindrical member 102 to that of the spiking components 22, 22' of Figures 11 and 12, but comprises a modified spiking element 80" extending perpendicularly from the exterior of the cylindrical member 102. The spiking element 80" may be considered as having a first part 410 and a second part 406, 408. The spiking element 80" has a rectangularly shaped cross-section (the first part 410 of the spiking element) with a free end provided with two sloping surfaces (only one 104" of which is shown in Figure 13) which extend from the major sides (as opposed to minor sides) of the rectangular cross-section and meet one another at an apex forming a ridge 108". The ridge 108" is therefore parallel to the major sides (as opposed to minor sides) of the spiking element 80" and, in use, the ridge 108 "pierces/perforates the seal, of a medicament compartment.

The ridge 108" extends beyond each of the minor sides 402, 404 by virtue of two projections 406, 408 projecting from the free end of the spiking elements 80" outwards along the major axis (of the rectangular shaped first part) and in a cantilevered arrangement from the exterior surface of the spiking element 80". The projections 406, 408 (the second part of the spiking element) thereby provide an over-hang at the free end of the spiking element 80". The dimension of the free end of the spiking element 80" along the major axis is therefore greater than at the root of the spiking element 80".

A flow path of rectangular cross-section extends through the spiking element 80" and opens onto the sloping surfaces to form an aperture 109" therein.

In use of the modified spiking element 80", the free end of said element 80" (and in particular the ridge 108") pierces the seal of a medicament compartment as the spiking element 80" is pressed into said compartment. The extension of the ridge 108" beyond the minor sides 402, 404 of the spiking element 80" (provided by the projections 406, 408) ensures that, as the over-hanging projections 406, 408 are moved past the container seal so that said seal locates nearer to the root 412 of the spiking element 80", the compartment seal is spaced from the minor sides 402, 404 so as to allow air to flow into said compartment between the seal and spiking element 80".

The width of the minor sides 402, 404 of the spiking element 80" (i.e. the dimension of the minor sides 402, 404 in the minor direction) is greater than that of the over-hanging projections 406, 408 and, accordingly, air flowing into a medicament compartment between the exterior of the spiking element 80" and the seal may readily flow around the over-hanging projections 406, 408. This is the case even if the ridge 108" is located in abutment with the bottom of a medicament compartment. Air flowing into a medicament compartment and then into the aperture 109" is denoted by arrow G in Figure 13. As the air flowing into the compartment changes direction to flow into the aperture 109", medicament within the compartment is entrained into the airflow and travels towards the inhaler outlet as denoted by arrow H.

It will be understood that the over-hanging projections 406, 408 may be added to the spiking elements of the spiking components 22, 22' shown in Figures 11 and 12 of the accompanying drawings.

The inhaler 1 may be sealed (i.e. unopenable) so that once the medicament within the cartridge 38 has been inhaled, the inhaler 1 must be discarded. Alternatively, the inhaler 1 may be provided with a lid, which allows access to the interior of the inhaler and thereby allows for a replacement of a used cartridge with a fresh cartridge. Accordingly, it will be understood that the inhaler 1 may incorporate a replaceable medicament cartridge so that, once the cartridge is exhausted, it can be replaced with a medicament cartridge which is fully loaded with medicament.

With regard to materials of manufacture, the top cover 6, middle cover 4, drive gear 33, counter and drive wheels 26, 30 and base 2 may be manufactured from Acrylonitrile Butadiene Styrene and other suitable materials already described in the specification. Also, the arm 90, spike component 22 (and any associated roller), and operating member 54 are manufactured from Acetyl co-polymer. Finally, the mouthpiece 8 and associated cover 10 are manufactured from polypropylene, and the medicament cartridge 38 is manufactured from polystyrene (206) or cyclic olefin copolymer.

The present invention is not limited to the specific embodiment described above. Alternative arrangements and suitable materials will be apparent to a reader skilled in the art. For example, in an alternative embodiment, a dose counter is not provided. In such an inhaler, the counter wheel 26 and window 24 will not be required and may be omitted. It will be understood therefore that the dose counter is an optional feature. It will also be appreciated that the spiking component 22 can be made integrally with the arm 90 so that the spiking component 22 and arm 90 form a unitary item.

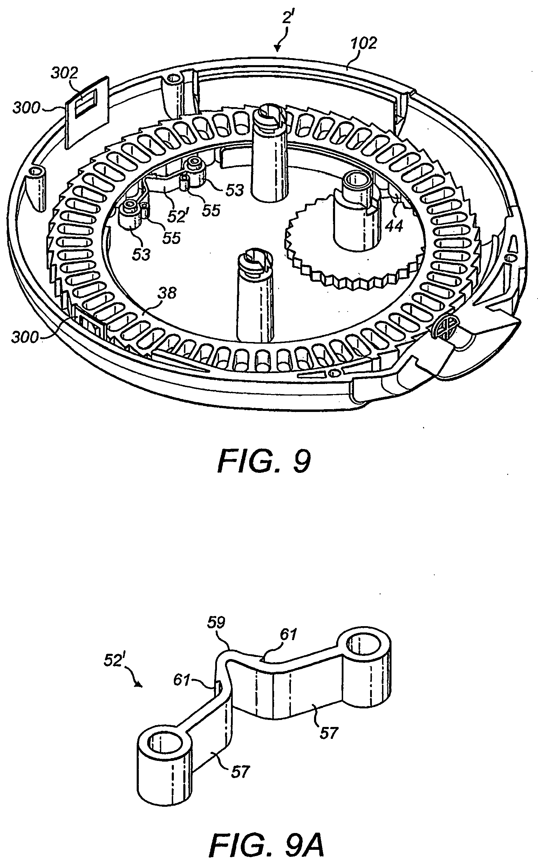

In a further alternative embodiment, a modified inhaler device is provided which is identical to the inhaler device of Figure 1 other than in that the base and associated deformable member have been modified. More specifically, with reference to Figures 9 and 9A, it will be seen that the modified base 2' of the modified inhaler is identical to the base 2 shown in Figure 6 other than that the modified base 2' is provided with two bosses 55 upstanding from the base 2'. Each boss 55 is located adjacent a different one of the two posts 53 upstanding from the base 2'. The position of each boss 55 relative to its associated post 53 is such that, when the deformable member 52' is fitted on to the posts 53, each leg 57 (see Figure 9A) abuts a different one of the two bosses 55. The abutment of the legs 57 with the bosses 55 is such that movement of the deformable member 52' towards the centre of the base 2' is resisted by the bosses 55. In essence, the force with which the resiliently deformable member 52' is sprung biased so as to be pressed into abutment with the cartridge 38 is greater in the modified base 2' than in the base 2 shown in Figure 6. This is particularly the case when the medicament cartridge 38 is rotated within the base 2' so that the element 59 (see Figure 9A) of the deformable member 52' is pressed inwardly by the teeth 44 of the cartridge 38 towards the centre of the base 2'. In this regard, it will be understood that, as the medicament cartridge 38 is rotated and the teeth 44 move laterally in abutment with the deformable member 52', the deformable member 52' is pressed inwardly as the element 59 rides over the apex of a tooth 44. In the base 2 of Figure 6, this inward movement results in a rotation of the legs 57 about the two upstanding posts 53. However, in the modified inhaler device, a rotation of the legs 57 about the upstanding posts 53 is limited by the abutment of the legs 57 with the bosses 55. There remains a bending of the legs 57 which allows the deformable member 52' to ride over the teeth 44 as the medicament cartridge 38 is rotated, however the restriction in the movement of the legs 57 as a result of the bosses 55 effectively increases the biasing force with which the element 59 is pressed against the cartridge 38 as the cartridge 38 is rotated. A consequence of this is that, once the deformable member 52' has ridden over the apex of a tooth 44, the member 52' tends to more forceably locate between said tooth 44 and an adjacent tooth 44 than is the case with the arrangement of base 2 in Figure 6. The forceful location of the deformable member 52' between neighbouring teeth 44 has a snap-action which tends to generate an audible click or other sound which assists in conforming to a user that the medicament cartridge 38 has been properly advanced.

The audible sound generated by the modified deformable member 52' is enhanced by the provision of an element 59 which, in the modified deformable member 52', has a thicker cross-section than in the deformable member 52 of Figure 6. The thicker cross-section is provided by means of two steps 61 (see Figure 9A). The steps 61 allow the element 59 to have a greater thickness (and, therefore, a greater stiffness) than the two legs 57, which are, of course, required to bend. The greater stiffness of the element 59 further contributes to increasing the force with which the deformable member 52' is pressed against the cartridge 38 as said cartridge 38 rotates within the base 2'. The greater stiffness of the element 59 also assists in generating a more audible sound as the deformable member 52' snaps back into position between two adjacent teeth 44.

The base 2' shown in Figure 9 also differs from the base 2 shown in Figure 6 in that the base 2' of Figure 9 is provided with two identical elements 300 extending from adjacent the upper perimeter edge 102 of the base 2'. The elements 300 have a generally rectangular shape with a rectangular aperture 302 provided therethrough. The elements 300 also have a resiliently deformable and elastic nature so that they may cooperate with protrusions (not shown) adjacent the lower perimeter edge 106 of the middle cover 4 (see Figure 7). The arrangement is such that, when the modified inhaler device is assembled, the perimeter surfaces 102, 106 mate with one another and, in so doing, the elements 300 deflect as they pass over said protrusions on the middle cover 4 and snap-fit over said protrusions so that each protrusion extends through a different aperture 302 of an element 300. In this way, the base 2' is resiliently clipped by means of the elements 300 and said protrusions to the middle cover 4. Each protrusion may have a rectangular shape similar to that of each aperture 302 and may also have curved or rounded surfaces so as to cam an associated element 300 over the protrusion as the base 2' and middle cover 4 are located in abutment with one another.

The modified base 2' may be used with any of the spiking components 22, 22', 22" mentioned above.

## Claims

1. An inhaler device (1) for dispensing doses of medicament, the inhaler comprising means for receiving a compartment (42) containing medicament; and medicament extraction facilitating means (22') for locating in a received compartment (42) and thereby allowing an extraction of medicament from said compartment (42); wherein the extraction facilitating means (22') comprises (i) a first fluid pathway extending therethrough for directing extracted medicament to an outlet of the inhaler and (ii) means which, in use, provides a second fluid pathway for directing fluid into said compartment past a sealing material of the compartment pierced by said extraction facilitating means; wherein the extraction facilitating means (22') comprises a member (102) defining a fluid pathway therein and an element (80) for piercing a sealing material of a received compartment (42) extending perpendicularly from an exterior of said member, the fluid pathway through the member (102) extending from a first end (120) of said member (102) to a second end (122) thereof, said element comprising a first part (103) provided with said first fluid pathway therein and a second part (110) providing said second fluid pathway, fluid communication being provided between the first fluid pathway and an outlet (124) at said second end (122); **characterised in that** the second part provides said second fluid pathway as a pathway extending though said part (110) and wherein the piercing element (80) comprises a ridge (108) for piercing a sealing material of a compartment (42), and wherein said piercing ridge (108) extends from the first part (103) to the second part (110).

2. An inhaler device according to claim 1, wherein the second part (110) comprises an inlet aperture (116) at an end thereof distal to an opposite end thereof which; in use, locates in a received compartment (42).

3. An inhaler device according to claim 2, wherein, in an extraction position of the medicament extraction facilitating means (22'), the inlet aperture (116) of the second part (110) locates on one side of pierced sealing material external to the compartment (42) and said opposite end of the second part (110) locates on the other side of the pierced sealing material internal to the compartment (42).

4. An inhaler device according to claim 2 or 3, wherein the second part (110) comprises an outlet aperture (114) at said opposite end thereof.

5. An inhaler device according to any of the preceding claims, wherein the piercing ridge (108) is an apex formed by the meeting of two surfaces (104, 106).

6. An inhaler device for dispensing multiple doses of medicament according to any of the preceding claims, the inhaler comprising means for receiving a medicament cartridge (38) comprising a plurality of compartments (42) containing medicament; and operating means moveable in first and second directions by a user; wherein the medicament extraction facilitating means (22') is movable from a retracted position to an extraction position in response to movement of the operating means in the first direction, the extraction position of the medicament extraction facilitating means (22') allowing extraction of medicament from said cartridge compartment (42) upon inhalation by a user, and the retracted position allowing advancement of the medicament cartridge (38), and wherein the medicament cartridge (38) is driven by the operating means so as to advance a cartridge compartment into a predetermined position relative to the medicament extraction facilitating means in response to movement of the operating means (22') in the second direction.

## Patentansprüche

1. Inhalatorvorrichtung (1) zum Ausgeben von Dosen eines Medikaments, wobei der Inhalator ein Mittel zur Aufnahme einer Medikament enthaltenden Kammer (42) und ein die Medikamententnahme ermöglichendes Mittel (22') zur Positionierung in einer aufgenommenen Kammer (42) und dadurch Zulassen einer Medikamententnahme aus der genannten Kammer (42) aufweist; wobei das die Entnahme ermöglichende Mittel (22') (i) eine erste Fluidbahn, die sich zum Lenken des entnommenen Medikaments zu einem Auslass des Inhalators dadurch hindurch erstreckt, und (ii) ein Mittel, das im Gebrauch eine zweite Fluidbahn zum Lenken von Fluid an einem Verschlussmaterial der Kammer vorbei, das von dem genannten die Entnahme ermöglichenden Mittel durchstochen wird, in die genannte Kammer hinein bereitstellt, aufweist; wobei das die Entnahme ermöglichende Mittel (22') ein Element (102), das darin eine Fluidbahn definiert, und ein Element (80) zum Durchstechen eines Verschlussmaterials einer aufgenommenen Kammer (42), das sich von einem Äußeren des genannten Elements lotrecht aberstreckt, aufweist, die Fluidbahn durch das Element (102) sich von einem ersten Ende (120) des genannten Elements (102) zu einem zweiten Ende (122) davon erstreckt, das genannte Element ein erstes Teil (103), das mit der genannten ersten Fluidbahn darin versehen ist, und ein zweites Teil (110), das die genannte zweite Fluidbahn bereitstellt, aufweist, zwischen der ersten Fluidbahn und einem Auslass (124) an dem genannten zweiten Ende (122) eine Fluidverbindung bereitgestellt wird; **dadurch gekennzeichnet, dass** das zweite Teil die genannte zweite Fluidbahn als eine Bahn bereitstellt, die sich durch das genannte Teil (110) erstreckt, und wobei das Stechelement (80) einen Grat (108) zum Durchstechen eines Verschlussmaterials einer Kammer (42) aufweist, und wobei der genannte Stechgrat (108) sich von dem ersten Teil (103) zu dem zweiten Teil (110) erstreckt.

2. Inhalatorvorrichtung nach Anspruch 1, wobei das zweite Teil (110) an einem Ende davon, das zu einem entgegengesetzten Ende davon distal ist, eine Einlassöffnung (116) aufweist, die im Gebrauch in einer aufgenommenen Kammer (42) liegt.

3. Inhalatorvorrichtung nach Anspruch 2, wobei in einer Entnahmeposition des die Medikamententnahme ermöglichenden Mittels (22') die Einlassöffnung (116) des zweiten Teils (110) auf einer Seite des durchstochenen Verschlussmaterials außerhalb der Kammer (42) liegt und das genannte entgegengesetzte Ende des zweiten Teils (110) auf der anderen Seite des durchstochenen Verschlussmaterials innerhalb der Kammer (42) liegt.

4. Inhalatorvorrichtung nach Anspruch 2 oder 3, wobei das zweite Teil (110) eine Auslassöffnung (114) an dem genannten entgegengesetzten Ende davon aufweist.

5. Inhalatorvorrichtung nach einem der vorhergehenden Ansprüche, wobei der Stechgrat (108) eine durch das Zusammentreffen zweier Oberflächen (104, 106) gebildete Spitze ist.

6. Inhalatorvorrichtung zur Ausgabe mehrerer Dosen eines Medikaments nach einem der vorhergehenden Ansprüche, wobei der Inhalator ein Mittel zur Aufnahme einer Medikamentenpatrone (38), die eine Vielzahl von Medikament enthaltende Kammern (42) aufweist, und ein Betätigungsmittel, das von einem Benutzer in einer ersten und einer zweiten Richtung bewegbar ist, aufweist; wobei das die Medikamententnahme ermöglichende Mittel (22') als Reaktion auf die Bewegung des Betätigungsmittels in der ersten Richtung von einer zurückgezogenen Position auf eine Entnahmeposition bewegbar ist, die Entnahmeposition des die Medikamententnahme ermöglichenden Mittels (22') bei Inhalation durch einen Benutzer die Entnahme von Medikament aus der genannten Patronenkammer (42) zulässt und die zurückgezogene Position die Voranbewegung der Medikamentpatrone (38) zulässt, und wobei die Medikamentpatrone (38) von dem Betätigungsmittel angetrieben wird, um eine Patronenkammer als Reaktion auf die Bewegung des Betätigungsmittels (22') in der zweiten Richtung in eine vorbestimmte Position relativ zu dem die Medikamententnahme ermöglichenden Mittel voranzubewegen.

## Revendications

1. Dispositif inhalateur (1) servant à distribuer des doses de médicament, l'inhalateur comprenant : un moyen de logement d'un compartiment (42) qui contient un médicament ; et un moyen facilitant l'extraction du médicament (22') destiné à être placé dans un compartiment (42) logé et à permettre ainsi une extraction de médicament à partir dudit compartiment (42) ; dans lequel le moyen facilitant l'extraction (22') comprend (i) un premier passage de fluide qui le traverse de façon à diriger le médicament extrait vers une sortie de l'inhalateur et (ii) un moyen qui, à l'utilisation, crée un deuxième passage de fluide de façon à diriger le fluide dans ledit compartiment au-delà d'un matériau d'étanchéité du compartiment percé par ledit moyen facilitant l'extraction ; dans lequel le moyen facilitant l'extraction (22') comprend un organe (102), à travers lequel un passage de fluide est défini, et un élément (80), servant à percer un matériau d'étanchéité d'un compartiment (42) logé, qui s'étend perpendiculairement depuis un extérieur dudit organe, le passage de fluide qui traverse l'organe (102) s'étendant d'une première extrémité (120) à une deuxième extrémité (122) dudit organe (102), ledit élément comprenant une première partie (103) pourvue dudit premier passage de fluide et une deuxième partie (110) qui réalise ledit deuxième passage de fluide, une liaison fluidique étant réalisée entre le premier passage de fluide et une sortie (124) au niveau de ladite deuxième extrémité (122) ; **caractérisé en ce que** la deuxième partie réalise ledit deuxième passage de fluide sous la forme d'un passage qui s'étend à travers ladite partie (110) et dans lequel l'élément de perçage (80) comprend une arête (108) destinée à percer un matériau d'étanchéité d'un compartiment (42), et dans lequel ladite arête de perçage (108) s'étend de la première partie (103) à la deuxième partie (110).

2. Dispositif inhalateur selon la revendication 1, dans lequel la deuxième partie (110) comprend à une de ses extrémités distale par rapport à son extrémité opposée un orifice d'entrée (116) qui, à l'utilisation, se trouve dans un compartiment (42) logé.

3. Dispositif inhalateur selon la revendication 2, dans lequel, dans une position d'extraction du moyen facilitant l'extraction du médicament (22'), l'orifice d'entrée (116) de la deuxième partie (110) se trouve d'un côté du matériau d'étanchéité percé à l'extérieur du compartiment (42) et ladite extrémité opposée de la deuxième partie (110) se trouve de l'autre côté du matériau d'étanchéité percé à l'intérieur du compartiment (42).

4. Dispositif inhalateur selon la revendication 2 ou la revendication 3, dans lequel la deuxième partie (110) comprend un orifice de sortie (114) au niveau de sa dite extrémité opposée.

5. Dispositif inhalateur selon l'une quelconque des revendications précédentes, dans lequel l'arête de perçage (108) est un sommet formé par la rencontre de deux surfaces (104, 106).

6. Dispositif inhalateur servant à distribuer de multiples doses de médicament selon l'une quelconque des revendications précédentes, l'inhalateur comprenant : un moyen de logement d'une cartouche de médicament (38) qui comprend une pluralité de compartiments (42) contenant un médicament ; et un moyen d'actionnement qui peut être déplacé dans une première et dans une deuxième direction par un utilisateur ; dans lequel le moyen facilitant l'extraction du médicament (22') peut être déplacé d'une position rentrée à une position d'extraction en réponse au déplacement du moyen d'actionnement dans la première direction, la position d'extraction du moyen facilitant l'extraction du médicament (22') permettant l'extraction de médicament à partir dudit compartiment de cartouche (42) lors d'une inhalation par un utilisateur, et la position rentrée permettant l'avance de la cartouche de médicament (38), et dans lequel la cartouche de médicament (38) est entraînée par le moyen d'actionnement de façon à faire avancer un compartiment de cartouche dans une position prédéterminée par rapport au moyen facilitant l'extraction du médicament en réponse au déplacement du moyen d'actionnement (22') dans la deuxième direction.
